# EUROPÄISCHE PATENTANMELDUNG

(11) **EP 0 898 947 A2**
(43) Veröffentlichungstag der Anmeldung: **03.03.1999**
(21) Anmeldenummer: 98113044.6
(22) Anmeldetag: 14.07.1998
(51) Int. Cl.: A61F 9/007

(54) **Verfahren und Vorrichtung zum Verbessern des Kammerwasserabflusses in einem Auge**

(30) Priorität: 15.08.1997 CH 1923/97; 10.03.1998 CH 574/98
(71) Anmelder: GRIESHABER & CO. AG SCHAFFHAUSEN, CH-8203 Schaffhausen (CH)
(72) Erfinder: Grieshaber, Hans R., 8200 Schaffhausen (CH); Stegmann, Robert, Prof. M.D., Pretoria 0181 (ZA)
(74) Vertreter: Althoff, Gerhard

(57) **Zusammenfassung**

Die Erfindung bezieht sich auf ein Verfahren zum Verbessern des Kammerwasserabflusses in einem Auge mit einem Schlemmschen Kanal, bei welchem Auge das von dem Ziliarkörper abgesonderte Kammerwasser über das nachgeordnete Kanalsystem abgeleitet wird, sowie auf eine Vorrichtung zur Aufrechterhaltung des Kammerwasserabflusses.

Mittels eines in den an mindestens einer Stelle mikrochirurgisch freigelegten Schlemmschen Kanal (15) injizierten Mediums, in Form einer hydrophilen Flüssigkeit oder eines biologisch verträglichen, gasförmigen Mediums oder eines Gemisches aus der hydrophilen Flüssigkeit und dem gasförmigen Medium, wird der Schlemmsche Kanal (15) durch den aufgebauten Druck lokal gedehnt. Mit einem anschliessend in den Innenraum (16) des Schlemmschen Kanals (15) implantierten Stützelement (35) wird dieser gestützt und permanent in geöffneter Stellung gehalten, wodurch ein ungehinderter Abfluss des Kammerwassers von dem Schlemmschen Kanal (15) über das nachgeordnete Kanalsystem (20) gewährleistet ist.

## Beschreibung

Die Erfindung bezieht sich auf ein Verfahren und eine Vorrichtung zum Verbessern des Kammerwasserabflusses in einem Auge, bei welchem das von dem Ziliarkörper abgesonderte Kammerwasser im Bereich des Kammerwinkels über das Trabekulargewebe in den Schlemmschen Kanal gelangt und von dort über das nachgeordnete natürliche Kanalsystem abgeleitet wird.

Für die Behandlung von Veränderungen des dem Schlemmschen Kanal vorgelagerten Trabekulargewebes, welche Veränderungen den Abfluss des Kammerwassers vollständig oder nur teilweise obstruieren, sind aus den Druckschriften US-A 5,360,399 und US-A 5,486,165 ein Verfahren sowie eine Vorrichtung zur Durchführung des Verfahrens bekannt, mittels welchem/welcher über eine in den Schlemmschen Kanal eingeführten Sonde ein vorzugsweise auf Hyaluronsäure basierendes Medium in Form einer hochviskosen, wässrigen Lösung derart injiziert wird, dass das Trabekulargewebe an einigen Stellen hydraulisch gedehnt und geöffnet wird, wobei die dabei entstehenden Öffnungen von der hochviskosen Lösung beschichtet werden und dadurch ein Verschliessen derselben für eine bestimmte Zeit weitgehend verhindert wird.

Obwohl in den vorstehend genannten Druckschriften das Injizieren eines geeigneten Mediums in den Schlemmschen Kanal als Mittel zum Öffnen desselben beschrieben ist, so besteht bei dem bekannten Verfahren nach wie vor die Unsicherheit, dass sich der Schlemmsche Kanal infolge verschiedener krankhafter Veränderungen wieder verschliesst. Der Abfluss des Kammerwassers über den Schlemmschen Kanal und über das nachgeordnete Kanalsystem ist somit infolge der Verformung des Schlemmschen Kanals erheblich eingeschränkt oder gar nicht mehr gewährleistet.

Aufgabe der vorliegenden Erfindung ist es, ein Verfahren anzugeben und eine Vorrichtung zu schaffen, mittels welchem/welcher eine einwandfreie und den Druck regulierende Zirkulation des Kammerwassers erreicht und der Abfluss desselben aus dem Auge dauerhaft aufrechterhalten wird.

Hinsichtlich des Verfahrens wird die Aufgabe dadurch gelöst, dass der an mindestens einer Stelle mikrochirurgisch freigelegte Schlemmsche Kanal in einer ersten Phase durch einen lokalen Druckaufbau gedehnt und anschliessend beziehungsweise in einer zweiten Phase durch geeignete und in den gedehnten Innenraum des Schlemmschen Kanals implantierte Mittel gestützt und dadurch permanent in gedehnter Stellung gehalten wird.

Die Aufgabe hinsichtlich der Vorrichtung wird dadurch gelöst, dass im Bereich des lokal gedehnten Teilstücks des Schlemmschen Kanals ein die Innenwand desselben stützendes sowie in axialer Richtung orientiertes Stützelement eingeführt und derart angeordnet ist, dass das Kammerwasser über das nachgeordnete natürliche Kanalsystem des Auges permanent ableitbar ist.

Weitere Vorteile, Merkmale und Einzelheiten der Erfindung ergeben sich aus den Patentansprüchen, der nachstehenden Beschreibung und der Zeichnung.

Ausführungsbeispiele der Erfindung werden nachstehend anhand der Zeichnung beschrieben. Es zeigt:
- **Fig.1**: einen schematisch und als Vertikalschnitt dargestellten vorderen Augenabschnitt;
- **Fig.2**: einen in schematischer Ansicht dargestellten Abschnitt des Auges mit eingeschnittener und aufgeklappter Lederhaut sowie teilweise freigelegtem Schlemmschen Kanal;
- **Fig.3**: einen in grösserem Massstab und schematisch dargestellten Abschnitt des Auges gemäss Fig.2 mit einer in den teilweise freigelegten Schlemmschen Kanal eingeführten Injektionssonde zur Dehnung des Kanals;
- **Fig.4**: den Abschnitt des Auges gemäss Fig.3 mit einem in das eine Teilstück des Schlemmschen Kanals implantierten Stützelement in Form einer zylindrischen Endoprothese;
- **Fig.5**: das räumlich sowie in grösserem Massstab dargestellte Stützelement gemäss dem in Fig.4 dargestellten ersten Ausführungsbeispiel;
- **Fig.6**: das in Ansicht sowie teilweise im Schnitt dargestellte Stützelement gemäss Fig.5;
- **Fig.7**: den Abschnitt des Auges gemäss Fig.3 mit einem in das andere Teilstück des Schlemmschen Kanals implantierten sowie als zweites Ausführungsbeispiel ausgebildeten Stützelement;
- **Fig.8**: das als zweites Ausführungsbeispiel in grösserem Massstab und teilweise im Schnitt dargestellte Stützelement gemäss Fig.7;
- **Fig.9**: ein drittes Ausführungsbeispiel des in den Schlemmschen Kanal zu implantierenden Stützelements;
- **Fig.10**: das gemäss der Linie X-X im Schnitt und in Seitenansicht dargestellte Stützelement gemäss Fig.9;
- **Fig.11**: ein viertes Ausführungsbeispiel des in den Schlemmschen Kanal zu implantierenden Stützelements; und
- **Fig.12**: ein weiteres Ausführungsbeispiel des in den Schlemmschen Kanal zu implantierenden Stützelements.

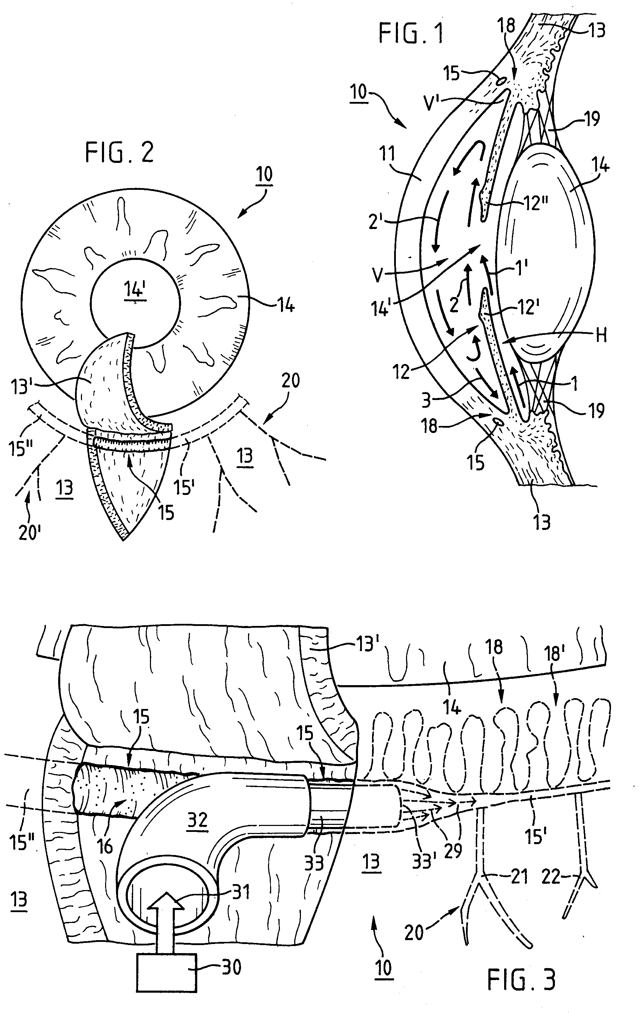

Fig.1 zeigt in grösserem Massstab den schematisch sowie als Vertikalschnitt dargestellten und in der Gesamtheit mit 10 bezeichneten vorderen Augenabschnitt und man erkennt die Hornhaut 11 (Cornea), die Regenbogenhaut 12 (Iris) mit den beiden Bereichen 12' und 12'', die Lederhaut 13 (Sklera), die Linse 14 mit der Pupille 14', die Zonulafasern 19 sowie den Schlemmschen Kanal 15 (Sinus venosus sclerae) und das demselben vorgelagerte Trabekulargewebe 18 (Trabeculum corneosclerale).

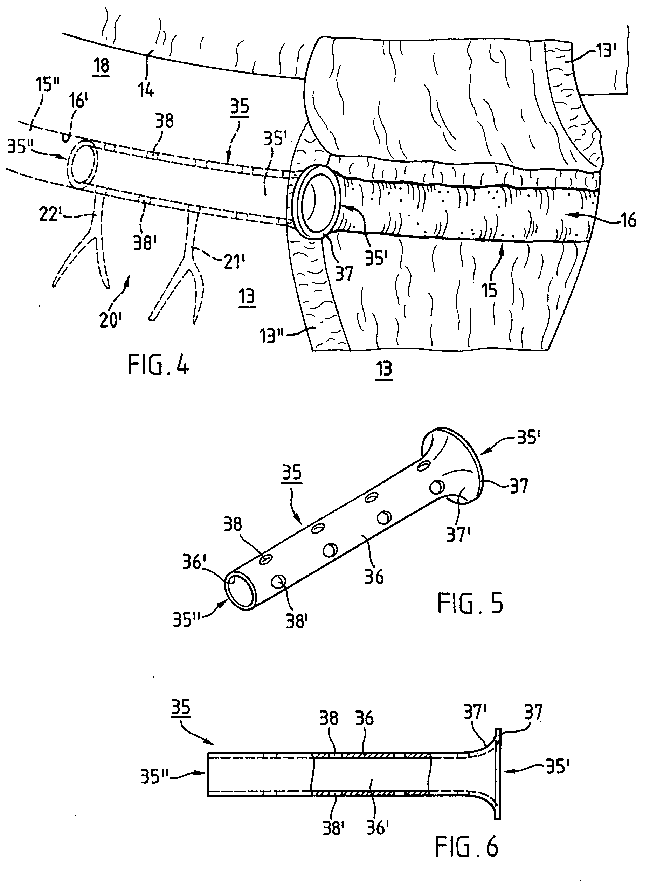

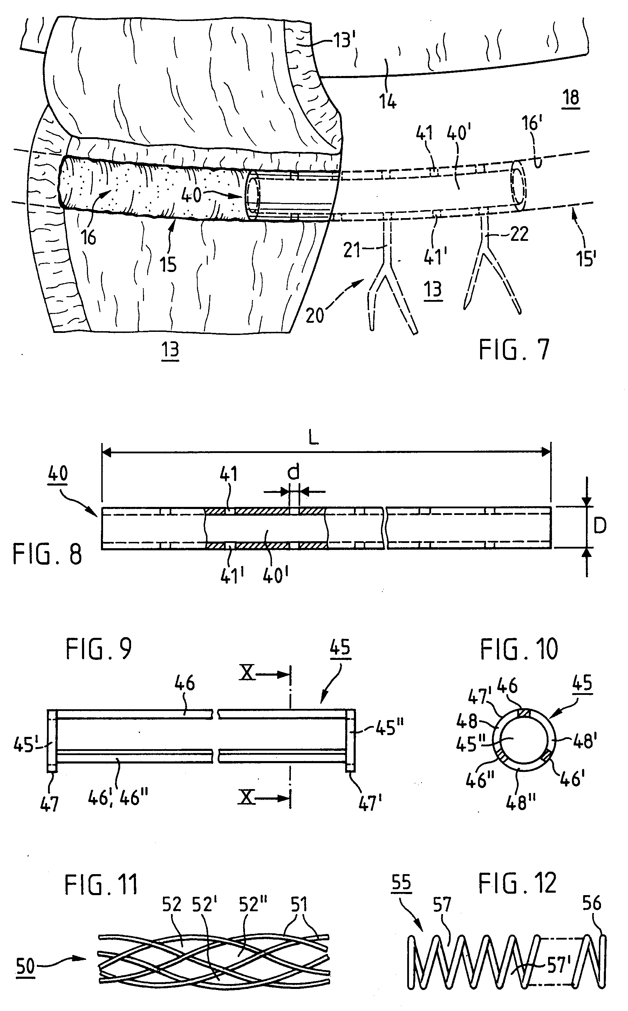

Bei einem gesunden Auge erfolgt der Abfluss des gemäss der Pfeile 1,1' und 2,2' von der Hinterkammer H zur Vorderkammer V zirkulierenden und sich ständig erneuernden Kammerwassers (Humor aquosus) im Kammerwinkel V' (Angulus iridocornealis) gemäss Pfeilrichtung 3 über das Trabekulargewebe 18 in den Schlemmschen Kanal 15 und von dort über die Sammelkanälchen und Kammerwasservenen 21',22' (Fig.4) beziehungsweise 21,22 (Fig.7) des nachgeordneten natürlichen Kanalsystems 20,20' (Fig.2,4,7) in das nicht dargestellte Venensystem. Hierbei wird von dem Widerstand des Venensystems der Abfluss des Kammerwassers derart reguliert, dass der Druck in einem von dem Augengewebe des betreffenden Auges tolerierten Bereich liegt.

Bei krankhaften Zuständen kann sich der Widerstand erhöhen, wobei einer der Faktoren im Schlemmschen Kanal liegt. Der Schlemmsche Kanal 15 kann sich derart verschliessen, dass der Abfluss des Kammerwassers behindert oder ausgeschlossen ist. Als Folge des erhöhten Widerstands steigt der Druck im Inneren des Auges so hoch an, dass in dem Auge die Durchblutung und in Folge davon die Funktion der Sehnerven eingeschränkt wird. Diese allgemein unter dem Begriff "Glaukom" bekannte Störung führt oftmals zum Erblinden eines oder beider Augen.

In Fig.2 ist das Auge 10 in schematischer Ansicht dargestellt und man erkennt die Linse 14 mit der Pupille 14', die teilweise dargestellte Lederhaut 13 sowie den teilweise dargestellten Schlemmschen Kanal 15 sowie ein Teilstück des damit in Verbindung stehenden natürlichen Kanalsystems 20,20' (Kammerwasser-Kanalsystem). Für den operativen Eingriff wird in einer ersten Phase, wie in Fig.2 schematisch dargestellt, die Lederhaut 13 mikrochirurgisch lamellar eingeschnitten und der äussere Teil als lappenförmiges Teilstück 13' zum teilweisen Freilegen des Schlemmschen Kanals 15 aufgeklappt. Für den weiteren operativen Eingriff wird das aufgeklappte Teilstück 13' mit hier nicht dargestellten Mitteln, wie Klammern oder dergleichen in dieser Stellung positioniert und gehalten.

In einer zweiten Phase wird, wie in Fig.3 schematisch dargestellt, eine röhrchenförmig ausgebildete und an einem schematisch dargestellten Anschlussstück 32 angeordnete Sonde 33 in den Innenraum 16 des Schlemmschen Kanals 15 eingeführt. Das Anschlussstück 32 steht über eine nicht dargestellte Zuführleitung mit einem schematisch dargestellten Injektionsgerät 30 in Verbindung. Mittels des Injektionsgerätes 30 wird gemäss Pfeilrichtung 31 über die am distalen Ende mit mindestens einer Austrittsöffnung 33' versehene röhrchenförmige Sonde 33 beispielsweise eine hydrophile Flüssigkeit 29 in das eine Teilstück 15' des Schlemmschen Kanals 15 injiziert. Mit der eingepressten hydrophilen Flüssigkeit 29 wird das in Fig.3 schematisch und weitgehend verschlossen dargestellte Teilstück 15' des Schlemmschen Kanals 15 hydraulisch gedehnt.

In Erweiterung oder Ergänzung des erfindungsgemässen Verfahrens kann in nicht näher dargestellter Weise mit einer vorzugsweise spiegelbildlich ausgebildeten und in den Schlemmschen Kanal 15 eingeführten Sonde das dem bereits behandelten Teilstück 15' gegenüberliegende Teilstück 15'' des Schlemmschen Kanals 15 analog behandelt und gedehnt werden. Weiterhin erkennt man in Fig.3 das dem Schlemmschen Kanal 15 vorgelagerte Trabekulargewebe 18 (Trabekelwerk) mit den schematisch dargestellten Gewebebälkchen 18'.

Bei der vorstehend beschriebenen Dehnung des Schlemmschen Kanals 15 werden gegebenenfalls in der Wandung entstehende Öffnungen (nicht dargestellt) gleichzeitig mit der beispielsweise injizierten, hydrophilen Flüssigkeit 29 beschichtet, so dass die an den Wandungen dieser Öffnungen in Form eines Films haften bleibende hydrophile Flüssigkeit eine den Abfluss des Kammerwassers behindernde lokale Gewebeverbindung verhindert.

An dieser Stelle wird darauf hingewiesen, dass anstelle der hydrophilen Flüssigkeit auch ein geeignetes, biologisch verträgliches gasförmiges Medium oder aber ein Gemisch aus der hydrophilen Flüssigkeit und dem gasförmigen Medium zum Dehnen des Schlemmschen Kanals verwendet werden können.

Wie in Fig.4 schematisch dargestellt, ist im Anschluss an die hydraulische oder pneumatische Dehnung sowie zur Optimierung einer dauerhaften Durchlässigkeit und Zirkulation des Kammerwassers in dem einen Teilstück 15'' des Schlemmschen Kanals 15 ein die Innenwand 16' stützendes Implantat eingesetzt. Als Implantat ist als erstes Ausführungsbeispiel ein mit einem länglichen Röhrchen 36 versehenes Stützelement 35 vorgesehen, welches mit dem einen, distalen Ende 35'' in den Schlemmschen Kanal 15 eingeführt ist. An dem anderen, proximalen Ende 35' ist das Stützelement 35 im dargestellten Ausführungsbeispiel mit einem an der Innenseite 13'' des Lederhaut-Einschnitts anliegenden Bund 37 versehen, durch welchen ein Verschieben des eingesetzten (implantierten) Stützelements 35 in dem Schlemmschen Kanal 15 verhindert wird. Das Röhrchen 36 ist weiterhin mit einer Anzahl in axialer Richtung sowie in Umfangsrichtung im Abstand verteilt angeordneten Durchtrittsöffnungen 38,38' versehen. Das Stützelement 35 wird, wie in Fig.4 schematisch dargestellt, vorzugsweise derart in dem Teilstück 15'' des Schlemmschen Kanals 15 plaziert und implantiert, dass mindestens eine der Durchtrittsöffnungen 38,38' mit den Kanälchen 21',22' des natürlichen Kanalsystems 20' in Verbindung steht.

Fig.5 zeigt das räumlich und Fig.6 das in Ansicht sowie teilweise im Schnitt dargestellte Röhrchen 36 des Stützelements 35 und man erkennt die verteilt angeordneten und mit dem Innenraum 36' in Verbindung stehenden Durchtrittsöffnungen 38, 38' sowie den im wesentlichen kreisringförmig ausgebildeten Bund 37. Im dargestellten Ausführungsbeispiel ist der zirkuläre Bund 37 beispielsweise mit einem kreisbogenförmigen Übergang 37' an dem Röhrchen 36 angeordnet, beispielsweise durch geeignete Mittel in Form eines nicht dargestellten Dorns aufgeweitet (kelchförmig) angeformt. Bei einem weiteren, nicht dargestellten Ausführungsbeispiel besteht zum besseren Einführen des Stützelements 35 in den Schlemmschen Kanal 15 zudem die Möglichkeit, dass das Röhrchen 36 ausgehend von dem Bund 37 bzw. von dem Übergang 37' in axialer Richtung bis zu dem distalen Ende 35'' konisch verjüngend ausgebildet ist.

Fig.7 zeigt das andere Teilstück 15' des Schlemmschen Kanals 15 mit dem als zweites Ausführungsbeispiel ausgebildeten und implantierten Stützelement 40. Das Stützelement 40 wird vorzugsweise so plaziert und implantiert, dass mindestens eine der Austrittsöffnungen 41,41', wie in Fig.7 schematisch dargestellt, mit den Kanälen 21,22 (Kanälchen) des natürlichen Kanalsystems 20 in Verbindung steht. Das in das Trabekulargewebe 18 eindringende Kammerwasser wird über den Schlemmschen Kanal 15 oder aber über den Innenraum 40' des Stützelements 40 sowie über die Öffnungen 41' und Kanälchen 21,22 des nachgeordneten natürlichen Kanalsystems 20 abgeleitet.

Fig.8 zeigt das in Ansicht und teilweise im Schnitt dargestellte zweite Ausführungsbeispiel des röhrchenförmig ausgebildeten Stützelements 40. Das zweite Stützelement 40 ist mit mehreren in axialer Richtung im Abstand zueinander sowie in Umfangsrichtung mit beliebig verteilt oder diametral zueinander angeordneten und mit dem Innenraum 40' in Verbindung stehenden Durchtrittsöffnungen 41,41' versehen.

In Fig.9 sowie in Fig.10 ist als drittes Ausführungsbeispiel das Stützelement 45 dargestellt, welches zwei in axialer Richtung beabstandete, kreisringförmig ausgebildete und je mit einer Öffnung 45',45'' versehene Endstücke 47,47' aufweist, zwischen welchen mindestens zwei, vorzugsweise aber drei in Umfangsrichtung im Abstand zueinander angeordnete und die Endstücke 47 und 47' miteinander verbindende Stege 46,46' und 46'' angeordnet sind. Bei dieser Variante dienen die zwischen den Stegen 46,46' und 46'' vorgesehenen Ausnehmungen 48,48' und 48'' jeweils als Durchtrittsöffnung für das im wesentlichen über die Öffnungen 45' und 45'' abzuführende Kammerwasser.

Fig.11 zeigt als viertes Ausführungsbeispiel das Stützelement 50, welches im wesentlichen als ein aus miteinander verbundenen, zweckmässig steif ausgebildeten Fäden 51 etwa schraubenlinienförmig gewundenes Netzgeflecht ausgebildet ist. Das Netzgeflecht kann beispielsweise aus relativ steifen Kunststoff- oder Metallfäden 51 oder biologischem Material hergestellt werden. Die einzelnen Fäden 51 (Filamente) des Netzgeflechts können dabei auch gegenläufig und schraubenlinienförmig gewunden miteinander verbunden sein. Bei dieser Variante dienen die zwischen den einzelnen Fäden 51 vorgesehenen Abstände 52,52' und 52'' jeweils als Durchtrittsöffnung für das Kammerwasser. Das Stützelement 50 kann derart ausgebildet sein, dass dieses zum Implantieren komprimiert wird und nach dem Implantieren selbsttätig im Innenraum 16 des Schlemmschen Kanals 15 expandiert.

Die miteinander verbundenen Metallfäden oder Filamente 51 des Stützelements 50 (Fig.11) sind vorzugsweise aus einer Nickel-Titan Legierung hergestellt. Diese Filamente 51 haben einen sogenannten Formgedächtniseffekt, welcher bewirkt, dass das als Netzgeflecht ausgebildete Stützelement 50 plastisch verformbar und bei entsprechender Erwärmung selbsttätig in die ursprüngliche Gestalt zurückführbar ist. Das Stützelement 50 mit dem thermischen Formgedächtnis (SHAPE MEMORY) hat den Vorteil, dass dieses beispielsweise unterhalb der normalen, menschlichen Körpertemperatur plastisch verformt mit kleinerem äusseren Durchmesser in den freigelegten Schlemmschen Kanal 15 einführbar ist und anschliessend infolge der normalen Körpertemperatur in die ursprüngliche Form oder Gestalt zurückgeführt wird.

In Fig.12 ist als weiteres Ausführungsbeispiel das Stützelement 55 dargestellt, welches beispielsweise aus einem einzigen, schraubenlinienförmig gewundenen Draht 56 aus Edelmetall, beispielsweise aus einem Silber-, Gold- oder Platindraht hergestellt ist. Bei dieser Variante dienen die zwischen den einzelnen Windungen vorgesehenen Abstände 57 und 57' jeweils als Durchtrittsöffnung für das Kammerwasser.

Die beispielsweise aus geeignetem, biologisch verträglichem Material hergestellten und röhrchenförmig oder spiralförmig

Die beispielsweise aus geeignetem, biologisch verträglichem Material hergestellten und röhrchenförmig oder spiralförmig ausgebildeten Stützelemente 35;40;45;50 oder 55 ermöglichen insbesondere aufgrund der eigenen Flexibilität eine optimale Anpassung an die natürliche Form des Schlemmschen Kanals 15. Die im wesentlichen hohlzylindrisch ausgebildeten Stützelemente 35;40;45;50 oder 55 können jedoch auch mit geeignetem Material beschichtet werden, wobei mit dem Beschichtungsmaterial gewünschte biologische Reaktionen erzeugt beziehungsweise unerwünschte biologische Reaktionen reduziert oder vollständig verhindert werden.

Bei einer nicht dargestellten Ausführungsvariante besteht jedoch auch die Möglichkeit, dass das Stützelement 35;40;45;50 oder 55 in Längsrichtung etwa bogenförmig ausgebildet ist. Bei einer weiteren, nicht dargestellten Variante besteht zudem die Möglichkeit, dass das Stützelement 35;40;45;50;55 von dem einen Ende in Richtung des anderen Endes und somit in Längsrichtung konisch verjüngend ausgebildet ist.

Zur Verdeutlichung der Abmessungen der einzelnen Stützelemente 35;40;45;50 und 55 und dessen diffizile Handhabung bei der Implantation in den gedehnten Innenraum 16 (Lumen) des Schlemmschen Kanals 15 (Fig.4,7) wird an dieser Stelle darauf hingewiesen, dass die Stützelemente beispielsweise eine Länge L=2,0 mm sowie einen äusseren Durchmesser D=0,2 mm aufweisen. Die in dem ersten und zweiten Ausführungsbeispiel gemäss Fig.5,6 und 8 in axialer Richtung sowie in Umfangsrichtung im Abstand zueinander angeordneten Durchtrittsöffnungen 38,38' oder 41,41' haben einen inneren, lichten Durchmesser d=0,18 mm. Die Stützelemente 35;40;45;50 und 55 sind jedoch nicht auf die vorstehend beispielsweise angegebenen Abmessungen begrenzt.

Ausführungsbeispiele zum Einführen des jeweiligen Stützelements:
**Variante I:** nach dem Dehnen wird das Injektionsgerät 30 mit der Sonde 33 aus dem Schlemmschen Kanal 15 herausgezogen und anschliessend das Stützelement 35;40;45;50 oder 55 mit geeigneten Mitteln in Form einer med. Zange (FORCEPS), Pinzette oder eines anderen chirurgischen Instruments manuell in den Innenraum 16 (Lumen) des Schlemmschen Kanals 15 eingeführt und plaziert (Fig.4,7);
**Variante II:** das Stützelement 35;40;45;50 oder 55 ist über eine trennbare Verbindung am distalen Ende der Sonde 33 des Injektionsgeräts 30 angeordnet und wird nach dem Dehnen des Schlemmschen Kanals 15 für die Implantation durch nicht dargestellte Mittel darin abgetrennt;
**Variante III:** das distale Ende der Sonde 33 des Injektionsgerätes 30 ist als abtrennbares Stützelement 35;40;45;50 oder 55 ausgebildet;
**Variante IV:** das im wesentlichen hohlzylindrische Stützelement 35;40;45;50 oder 55 ist auf das distale Ende der Sonde 33 derart aufgeschoben, dass nach dem Dehnen des Schlemmschen Kanals 15 das Stützelement 35;40;45;50 oder 55 durch geeignete Mittel in axialer Richtung relativ zu der Sonde 33 in den Innenraum 16 (Lumen) des Schlemmschen Kanals 15 geschoben und plaziert wird.

Weitere zweckmässige und im Rahmen der Erfindung liegende Varianten zum Implantieren des Stützelements 35;40;45;50 oder 55 in den Innenraum 16 (Lumen) des Schlemmschen Kanals 15 sind ebenfalls möglich.

Die Erfindung ist nicht auf die vorstehend beschriebenen Ausführungsbeispiele der einzelnen Stützelemente 35;40;45;50 oder 55 beschränkt. Weitere zweckmässige Ausgestaltungen der im wesentlichen als Implantat ausgebildeten Stützelemente, ohne dabei den Grundgedanken der Erfindung zu verlassen, sind ebenfalls möglich. Die vorstehend im einzelnen beschriebenen und dargestellten Stützelemente werden häufig auch als Endoprothesen bezeichnet. Als besonders vorteilhaft wird die Kombination des hydraulischen Dehnens und Öffnens des in Fig.3 schematisch und weitgehend verschlossen dargestellten Schlemmschen Kanals 15 mit der anschliessenden Implantation des entsprechend ausgebildeten Stützelements 35;40;45;50 oder 55, insbesondere des flexibel ausgebildeten Stützelements angesehen.

Mit dem Stützelement 35 oder 40 wird der Innenraum 16 des Schlemmschen Kanals 15 dauerhaft offengehalten, wobei das Stützelement 35 oder 40 beispielsweise so plaziert wird, dass mindestens eine der Austrittsöffnungen 38,38' oder 41,41', wie in Fig.4 und Fig.7 schematisch dargestellt, mit den Kanälchen 21',22' oder 21,22 des nachgeordneten Kanalsystems 20' oder 20 in Verbindung steht. Das in das Trabekulargewebe 18 eindringende Kammerwasser wird über den Schlemmschen Kanal 15 oder aber über den Innenraum 36' oder 40' des Stützelements 35 oder 40 und über die Öffnungen 38' oder 41' und Kanälchen 21',22' oder 21,22 des nachgeordneten natürlichen Kanalsystems 20' oder 20 abgeleitet.

Es wird weiterhin darauf hingewiesen, dass in den Innenraum 16 des Schlemmschen Kanals 15 mindestens ein an der Innenwand 16' des Schlemmschen Kanals 15 abstützend anliegendes sowie in axialer Richtung orientiertes Stützelement 35;40;45; 50;55 implantiert wird. Bei Bedarf besteht jedoch auch die Möglichkeit, dass in Abhängigkeit des verformten und verstopften Schlemmschen Kanals 15 zwei oder mehrere Stützelemente implantiert werden. Hierbei ist es vorteilhaft, wenn das jeweils implantierte Stützelement eine Verbindung des Schlemmschen Kanals 15 mit mindestens einem Kanal 21,22 oder 21',22' des nachgeordneten natürlichen Kanalsystems 20 oder 20' gewährleistet.

## Patentansprüche

1. Verfahren zum Verbessern des Kammerwasserabflusses in einem Auge, bei welchem das von dem Ziliarkörper abgesonderte Kammerwasser im Bereich des Kammerwinkels über das Trabekulargewebe (18) in den Schlemmschen Kanal (15) und von dort über das nachgeordnete natürliche Kanalsystem abgeleitet wird, **dadurch gekennzeichnet,** dass der an mindestens einer Stelle mikrochirurgisch freigelegte Schlemmsche Kanal (15) in einer ersten Phase durch einen lokalen Druckaufbau gedehnt und anschliessend beziehungsweise in einer zweiten Phase durch geeignete und in den gedehnten Innenraum (16) des Schlemmschen Kanals (15) implantierte Mittel gestützt und dadurch permanent in gedehnter Stellung gehalten wird.

2. Verfahren nach Anspruch 1, **dadurch gekennzeichnet,** dass der freigelegte Schlemmsche Kanal (15) mit einer in den Innenraum (16) desselben injizierten, hydrophilen Flüssigkeit gedehnt wird.

3. Verfahren nach Anspruch 1, **dadurch gekennzeichnet,** dass der freigelegte Schlemmsche Kanal (15) mit einem in den Innenraum (16) desselben injizierten, gasförmigen und biologisch verträglichem Medium gedehnt wird.

4. Verfahren nach den Ansprüchen 2 und 3, **dadurch gekennzeichnet,** dass der freigelegte Schlemmsche Kanal (15) mit in den Innenraum (16) desselben injiziertem Gemisch aus der hydrophilen Flüssigkeit und dem gasförmigen Medium gedehnt wird.

5. Verfahren nach Anspruch 1, **dadurch gekennzeichnet,** dass in das gedehnte Teilstück (15',15'') des Schlemmschen Kanals (15) mindestens ein die Innenwand (15') desselben permanent abstützendes sowie in axialer Richtung orientiertes Stützelement (35;40;45;50;55) implantiert wird.

6. Verfahren nach den Ansprüchen 1 und 5, **dadurch gekennzeichnet,** dass das mit dem distalen Ende (35'') in das gedehnte Teilstück (15',15'') des Schlemmschen Kanals (15) eingeführte Stützelement (35) durch am proximalen Ende (35') desselben angeordnete Mittel an der Innenwand (13'') des Einschnitts anliegend gehalten wird.

7. Verfahren nach den Ansprüchen 1 und 5, **dadurch gekennzeichnet,** dass in das gedehnte Teilstück (15',15'') des Schlemmschen Kanals (15) ein mindestens im äusseren Durchmesser plastisch verformbares und infolge eines thermischen Formgedächtnisses in die ursprüngliche Form zurückführbares Stützelement (50) implantiert wird.

8. Verfahren nach Anspruch 7, **dadurch gekennzeichnet,** dass das Stützelement (50) unterhalb der normalen, menschlichen Körpertemperatur plastisch verformt und nach dem Implantieren infolge der Körpertemperatur und des Formgedächtnisses (SHAPE MEMORY) in die ursprüngliche Form zurückgeführt wird.

9. Verfahren nach den Ansprüchen 1 und 5, **dadurch gekennzeichnet,** dass der Schlemmsche Kanal (15) an mindestens zwei in Umfangsrichtung im Abstand zueinander angeordneten Teilstücken (15',15'') gedehnt wird, in welche jeweils das Stützelement (35;40;45;50;55) implantiert wird.

10. Verfahren nach den Ansprüchen 1,5 und 9, **dadurch gekennzeichnet,** dass das Stützelement (35;40;45;50;55) mit daran verteilt angeordneten und mit dem Innenraum desselben in Verbindung stehenden Durchtrittsöffnungen oder dergleichen mit dem natürlichen Kanalsystem (20;20') in Verbindung stehend in dem Schlemmschen Kanal (15) implantiert wird.

11. Verfahren nach den Ansprüchen 1 bis 10, **dadurch gekennzeichnet,** dass zu beiden Seiten des mikrochirurgisch freigelegten und gedehnten Teilstücks (15',15'') des Schlemmschen Kanals (15) jeweils ein Stützelement (35;40;45;50;55) implantiert wird.

12. Vorrichtung zur Durchführung des Verfahrens nach Anspruch 1, bei welchem der an mindestens einer Stelle freigelegte Schlemmsche Kanal (15) mittels eines von einem eingeführten Injektionsgerät (30) injizierten Mediums gedehnt ist, **dadurch gekennzeichnet,** dass im Bereich des lokal gedehnten Teilstücks (15',15'') des Schlemmschen Kanals (15) ein die Innenwand (16') stützendes sowie in axialer Richtung desselben orientiertes Stützelement (35;40;45;50;55) eingeführt und derart angeordnet ist, dass das Kammerwasser über das nachgeordnete natürliche Kanalsystem (20;20') des Auges (10) permanent ableitbar ist.

13. Vorrichtung nach Anspruch 12, **dadurch gekennzeichnet,** dass das als längliches Röhrchen (36) ausgebildete Stützelement (35) an dem dem distalen Ende (35'') gegenüberliegenden proximalen Ende (35') mit einem in bezug auf das Röhrchen (36) radial nach aussen konisch erweiternd ausgebildeten Anlagebund (37), vorzugsweise mit einem angeformten Anlagebund (37) versehen ist.

14. Vorrichtung nach Anspruch 12, **dadurch gekennzeichnet,** dass das Stützelement (35;40;45;50;55) in axialer Richtung analog dem Innenraum (16) des Schlemmschen Kanals (15) etwa bogenförmig oder selbsttätig bogenförmig verformbar ausgebildet ist.

15. Vorrichtung nach Anspruch 12, **dadurch gekennzeichnet,** dass das Stützelement (35;40;45;50;55) in Längsrichtung von dem einen proximalen Ende in Richtung des anderen distalen Endes konisch verjüngend ausgebildet ist.

16. Vorrichtung nach Anspruch 12, **dadurch gekennzeichnet,** dass das röhrchenförmige Stützelement (35;40) mit mehreren in Umfangs- und Längsrichtung im Abstand verteilt zueinander angeordneten und mit dem Innenraum (36',40') in Verbindung stehenden Durchtrittsöffnungen (38,38';41,41') versehen ist.

17. Vorrichtung nach Anspruch 12, **dadurch gekennzeichnet,** dass das Stützelement (45) zwei in axialer Richtung beabstandete, kreisringförmig ausgebildete sowie mit Durchtrittsöffnungen (45';45'') versehene Endstücke (47,47') aufweist, zwischen welchen mindestens zwei, vorzugsweise aber drei in Umfangsrichtung im Abstand zueinander angeordnete Stege (46, 46',46'') angeordnet sind.

18. Vorrichtung nach Anspruch 12, **dadurch gekennzeichnet,** dass das Stützelement (50) aus miteinander verbundenen Fäden (51) als hohlzylindrisches und mit Durchtrittsöffnungen (52, 52',52'') versehenes Netzgeflecht ausgebildet ist.

19. Vorrichtung nach Anspruch 18, **dadurch gekennzeichnet,** dass das Netzgeflecht des Stützelements (50) plastisch verformbar und durch Erwärmung in die ursprüngliche Formgebung zurückführbar ist.

20. Vorrichtung nach den Ansprüchen 18 und 19, **dadurch gekennzeichnet,** dass die Fäden (51) des Netzgeflechts aus einer Nickel-Titan-Legierung mit thermischem Formgedächtniseffekt hergestellt sind.

21. Vorrichtung nach den Ansprüchen 18 und 19, **dadurch gekennzeichnet,** dass die Fäden (51) des Netzgeflechts aus Kunststoff mit thermischem Formgedächtniseffekt hergestellt sind.

22. Vorrichtung nach den Ansprüchen 18 bis 21, **dadurch gekennzeichnet,** dass das Stützelement (50) aus einfach oder gegenläufig gewundenen Fäden (51) als hohlzylindrisches Netzgeflecht ausgebildet ist.

23. Vorrichtung nach den Ansprüchen 18 bis 21, **dadurch gekennzeichnet,** dass die Fäden (51) des Stützelements (50) in Form eines schraubenlinienförmig gewundenen Netzgeflechts miteinander verbunden sind.

24. Vorrichtung nach Anspruch 18, **dadurch gekennzeichnet,** dass das als hohlzylindrisches Netzgeflecht ausgebildete Stützelement (50) selbstexpandierend ausgebildet ist.

25. Vorrichtung nach Anspruch 18, **dadurch gekennzeichnet,** dass das netzförmige Stützelement (50) aus mehreren miteinander gewundenen Metalldrähten hergestellt ist.

26. Vorrichtung nach Anspruch 12, **dadurch gekennzeichnet,** dass das Stützelement (55) in Form einer Schraubenfeder aus einem einzigen, schraubenlinienförmig gewundenen Draht (56) hergestellt ist.

27. Vorrichtung nach Anspruch 12, **dadurch gekennzeichnet,** dass das etwa hohlzylindrische Stützelement (35;40;45;50;55) aus biokompatiblem Material, beispielsweise aus geeignetem Kunststoff, aus nichtrostendem Stahl, aus einem Edelmetall, wie beispielsweise Silber, Gold oder Platin oder einem biologischen Material hergestellt ist.

28. Vorrichtung nach Anspruch 12, **dadurch gekennzeichnet,** dass das etwa hohlzylindrische Stützelement (35;40;45;50;55) zur Erzeugung einer gewünschten beziehungsweise zur Vermeidung einer unerwünschten biologischen Reaktion mit geeignetem Material beschichtet ist.

29. Vorrichtung nach Anspruch 12, **dadurch gekennzeichnet,** dass das etwa hohlzylindrische Stützelement (35;40;45;50;55) zum selbsttätigen Anpassen an den Innenraum (16) des Schlemmschen Kanals (15) in Richtung der theoretischen Längsachse flexibel ausgebildet ist.

30. Vorrichtung nach Anspruch 12, **dadurch gekennzeichnet,** dass das etwa hohlzylindrische Stützelement (35;40;45;50;55) durch eine trennbare Verbindung an dem Injektionsgerät (30) angeordnet ist.

31. Vorrichtung nach Anspruch 12, **dadurch gekennzeichnet,** dass das Injektionsgerät (30) mit einer Sonde (33) versehen ist, welche als abtrennbares und in den Innenraum (16) des Schlemmschen Kanals (15) zu implantierendes Stützelement (35; 40;45;50;55) ausgebildet ist.

32. Vorrichtung nach den Ansprüchen 12 und 31, **dadurch gekennzeichnet,** dass das etwa hohlzylindrische Stützelement (35;40;45;50;55) auf die Sonde (33) des Injektionsgerätes (30) aufgeschoben und durch eine axiale Bewegung der Sonde (33) relativ zu dem Stützelement (35;40;45;50;55) oder umgekehrt in das gedehnte Teilstück (15',15'') des Schlemmschen Kanals (15) lagestabil einsetzbar ist.
